(19) Europäisches Patentamt
European Patent Office
Office européen des brevets

(11) EP 4 311 478 A1

(12) EUROPEAN PATENT APPLICATION

(43) Date of publication:
**31.01.2024 Bulletin 2024/05**

(21) Application number: **22187471.2**

(22) Date of filing: **28.07.2022**

(51) International Patent Classification (IPC):
***A61B 5/00*** *(2006.01)* ***A61B 5/103*** *(2006.01)*
***G06T 7/90*** *(2017.01)* ***G01N 21/00*** *(2006.01)*

(52) Cooperative Patent Classification (CPC):
**A61B 5/0071; A61B 5/1032; A61B 5/444; A61B 5/4887; A61B 5/7267; G01N 21/6486; G06T 7/90;** A61B 5/443; A61B 2576/00; G01N 2021/6417

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Magnosco GmbH**
**12489 Berlin (DE)**

(72) Inventors:
- **SZYC, Lucasz**
  **12347 Berlin (DE)**
- **DRONNIK, Eduard**
  **12529 Schönefeld (DE)**

(74) Representative: **Gulde & Partner**
**Patent- und Rechtsanwaltskanzlei mbB**
**Wallstraße 58/59**
**10179 Berlin (DE)**

Remarks:
Amended claims in accordance with Rule 137(2) EPC.

# (54) METHOD FOR OBTAINING DERMATOFLUOROSCOPY DATA OPTIMIZED FOR CLASS SEPARABILITY

(57) The present invention concerns a method for obtaining dermatofluoroscopy data, the method comprising: illuminating a region of interest on a skin lesion using a predetermined light spectrum; obtaining a polarized dermoscopy, PD, image using a camera, wherein each pixel of the PD image comprises a brightness value and/or at least one color value; defining a first scanning grid and mapping the first grid to the PD image; determining, for each grid point of the first grid, based on the brightness value and/or at least one color value, a first likelihood of detecting low-noise two photon fluorescence, TPF, spectra in the vicinity of the grid point; determining a second grid based on the calculated first likelihoods for the first grid points; performing a dermatofluoroscopic scan, including the detection of TPF spectra, on the grid points of the second grid; and determining a second likelihood for each grid point of the second grid based on the dermatofluoroscopic scan, the second likelihood being associated with malignancy.

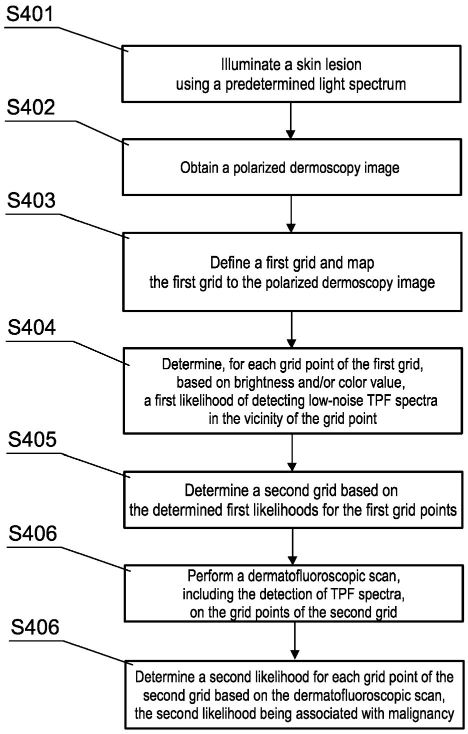


Fig. 4

EP 4 311 478 A1

## Description

### TECHNICAL FIELD

The present invention relates to a method for performing a dermatofluoroscopic scan, using a suitable scanner device, on a spatially arranged grid of scan points in an area of interest of a person's skin. Particularly, the grid is arranged under the constraint of minimizing the scan duration while providing low-noise dermatofluoroscopic data.

### BACKGROUND OF THE INVENTION

The term dermatofluoroscopy refers to a method and/or an apparatus designed and suitable for early diagnostics of cutaneous melanoma. Therein, a dermatofluoroscopic method and/or apparatus may utilize the following modalities: a two-photon fluorescence (TPF) spectroscopy module as described e.g., in US 7888659 B2 and a dermoscope module utilizing homogeneous illumination with polarized (PD, Polarized Dermoscopy) RGB, NIR and/or white light.

Retrospective studies have carried out a laser scan at spots on a regular grid of 0.2 mm steps (typically the laser has a focus of about 0.02 mm radius, equal to 1/10 step size) collecting dermatofluoroscopic signals. In this way, several hundred spectra are collected per lesion/sample, with the number of scan points growing as the square of the lesion radius. In addition, a dermoscopic-quality, polarized RGB/NIR or white-light image is used, together with the dermatofluoroscopic spectra, for classification of the measured skin tumour as being either malignant or benign.

The dermatofluoroscopic scan time is a linear function of the area of skin lesion of interest and the density of scan points. The longer the scan, the lower is usually the usability as the patient must be still for the entire duration of the measurement.

The ability to detect fluorescence signals of melanin from fluorophore mixtures in solid samples using the dermatofluoroscopy method, as e.g., described in US7888659B2, is the key feature of the in-vivo, noninvasive differential diagnosis of pigmented cutaneous melanoma and melanocytic (dysplastic) nevus. In this context, a skin tumor with a diameter of 10 mm is considered large, while not being uncommon. With the aforementioned regular grid of 0.2 mm spacing between the scan points, the total number of spectra collected would be more than 1800. As the key of dermatofluoroscopy is the selective detection of the ultra-weak fluorescence of skin melanins, the time required to collect it (i.e. photoexcitation, acquisition, detection) from one scan point cannot be reduced below 100 ms, with a typical value of 1000 ms. With 1000 ms per scan point and a 10 mm skin lesion, the pure measurement time would be at least 30 minutes.

Since the skin tumors represent the class of skin diseases with high histological and cellular heterogeneity, the entire lesion may not necessarily contain information about its malignant or benign nature. Therefore, any scanning method that uses focused light for differential diagnosis between skin cancer and benign tumors may suffer from a certain amount of spatially resolved signals collected in biologically irrelevant areas. Such signals could be considered as noise.

The high number of scan points of current dermatofluoroscopy methods hence disadvantageously increases the overall scan time while at the same time potentially contributing to noise.

It is therefore an object of the present invention to obviate or reduce at least some of the drawbacks of the prior art and to provide a method for obtaining dermatofluoroscopic data which can reduce the scan time while providing high-quality, i.e., low-noise, data acquisition with the appropriate instrumentation.

### DESCRIPTION OF THE INVENTION

According to a first aspect of the invention, there is provided a method for obtaining dermatofluoroscopy data, the method comprising: illuminating a region of interest on a skin lesion using a predetermined light spectrum; obtaining a polarized dermoscopy, PD, image using a camera, wherein each pixel of the PD image comprises a brightness value and/or at least one color value; defining a first grid and mapping the first grid onto the PD image; determining, for each grid point of the first grid, based on the brightness value and/or the at least one color value, a first likelihood of detecting low-noise two photon fluorescence, TPF, spectra in the vicinity of the grid point; determining a second grid based on the determined first likelihoods for the first grid points; performing a dermatofluoroscopic scan, including the detection of TPF spectra, on the grid points of the second grid; and determining a second likelihood for each grid point of the second grid based on the dermatofluoroscopic scan, the second likelihood being associated with malignancy.

Polarized Light Dermatoscopy, PD, is a method known in the art that uses the polarized light source and the detector (e.g., a CCD camera or an eye). Such instruments typically include one or more illumination sources, such as light-emitting diodes, to provide the homogeneous intense illumination required to obtain a high-quality PD image of a region of interest. Light reflected back from the skin surface to the detector must first pass through a polarizer that blocks all photons of the original polarization of the light source (cross-polarization). Consequently, unwanted surface glare in PD is significantly reduced. If the originally polarized light undergoes sufficient scattering events in the skin ("polarization randomization"), back-reflected "deep penetrating light" can pass through the cross-polarizing filter and enter the eye or another detector, allowing visualization of dermoscopic structures of the epidermis stratum granulosum through the dermal-epidermal junction to the superficial dermis. In other words, PD is "blind" to back-reflected light from the superficial layers of the epidermis.

In order to collect two-photon excited (TPF) signals, the laser light is tightly focused into a 20 to 40 $\mu$m spot, approx. 100 $\mu$m in the skin depth, which enables an electronic excitation of the skin fluorophores of all epidermal layers as well as the upper dermis layers.

It is clear to a person skilled in the art that a skin lesion is located in a region of interest and typically a dermatofluoroscopy scan is applied to an area at least slightly larger than the skin lesion under evaluation. In relation to the PD image the first grid will typically be a grid of regular intervals between grid points such as 0.2 mm but it may be non-regular and even if it is regular the spacing may be different. By mapping the first grid to the PD image, information is obtained on PD image pixels corresponding to the first grid. Such pixels have corresponding color and/or brightness. Using this information, a first probability of detecting low-noise two-photon fluorescence (TPF) spectra in the vicinity of the grid point is determined for each grid point of the first grid based on the brightness value and/or the at least one color value. The first likelihood is preferably determined based on past TPF scans that provide information mapping the brightness value and/or the at least one color value to a likelihood of detecting low-noise two photon fluorescence, TPF, spectra in the vicinity of the grid point.

According to the present disclosure the predetermined light spectrum is preferably at least one of red, green, blue, white, infrared, and near infrared.

According to the present disclosure the number of grid points of the second grid is preferably equal to or lower than the number of grid points of the first grid. The grid optimization prior to the TPF scan has several advantages over the existing regular scan grid with the fixed distance (e.g. 0.2 mm) between the scan points. There is obtained improved usability of the device as well as less noise in collected data. Specifically, there may be achieved a reduced measurement time (improved usability, less stress to the examined person). Additionally, improved data collection allows for enhanced class separability, and consequently, for better accuracy of the process. The class separability is preferably defined as the overlap area of two class-conditional distributions.

The step of determining a second grid based on the determined first likelihoods for the first grid points is aimed at using past TPF scans to optimize the grid arrangement such that improved scan results may be obtained and/or shorter scan may be achieved due to a lower number of scan points. In order to optimize the second grid, grid points may be removed in areas of lower interest or in areas where a given plurality of grid points does not improve probability of correct classification after a final scan. Correspondingly, grid points may be added in areas of high interest or in areas where additional grid points are expected to improve probability of correct classification after the final scan. Preferably, the second grid is a subset of the first grid.

When the second grid is determined, a dermatofluoroscopic scan is performed including the detection of TPF spectra at the grid points of the second grid. Thus, the TPF scan is performed on an optimized grid obtained based on a PD image and previous TPF scan data. Finally, to use the actual scanned data and results, the method involves determining a second likelihood for each grid point of the second grid based on the dermatofluoroscopic scan, the second likelihood being associated with malignancy.

According to an embodiment, the second likelihood is preferably determined using a malignancy model trained by a (first) machine learning, ML, algorithm and associating dermatofluoroscopic scan data, including at least TPF spectra data, with a class label related to malignancy. In an embodiment this process is preferably based on past scans that are analyzed and described in relation to diagnosed malignancy. Therefore, such a class label related to malignancy may be a Boolean indication of malignant/non-malignant. Such an assignment of the class label to dermatofluoroscopic scan data takes place on the basis of dermatofluoroscopic spectra and preferably at least one color value and/or at least one brightness value of the pixel/point.

In an embodiment, the (first) ML model may be trained on the hyperimage trainset of spatially resolved dermatofluoroscopic signals and RGB/white-NIR images. The model generates a malignancy score; it is a 1-D measure that the lesion is malignant (e.g., between 0 and 1). The ML model is preferably optimized in such a way that a high area under receiver operating characteristic (AUROC) and high sensitivity (true positive rate at a certain fixed threshold/"cut-off") are obtained for early melanoma detection.

Preferably, a low-noise TPF spectrum provides class separability above a predefined threshold when input to the malignancy model, where class separability is related to at least two classes of mutually exclusive class labels. Herein it is defined, that preferably two opposite classes are considered, i.e. a malignant class and a non-malignant class. In such cases, the lower the noise in the collected data, the higher the separability between the opposite classes. As class separability is preferably defined as the overlap area of two class-conditional distributions, the threshold - in one exemplary preferred embodiment - is related to an integral value of the overlap area of two class-conditional distributions. In general, a class separability is improved, when the overlap area of two class-conditional distributions is reduced and hence the threshold might be defined based on an inverse value of a numeric value of said integral value of said overlap.

According to an embodiment combinable with any of the preceding embodiments, the first likelihood is determined by a grid model and/or a look up table, LUT, both associating image features defined by a brightness value and/or the at least one color value with a likelihood of detecting a low-noise TPF spectra.

Preferably, the grid model is determined in a calibration phase based on PD image data, TPF spectra data and class separability data. This calibration phase also pertains to a second aspect of the invention as described in more detail below. Further preferred, the grid model is trained in the calibration phase by a (second) machine learning, ML, algorithm associating, for each grid point of a plurality of previously scanned grids, PD image data, TPF spectra data and class separability data. One or more sets of data are preferably collected during past clinical studies (spectra and corresponding images with correct class labels). In short, the (second) ML-algorithm analyses the dermoscopic-quality image of the lesion of interest by searching for image features (colors, pigmentation patterns, vessels, etc.) that corresponds to high probability of measuring the low-noise spectra (i.e. the spectra of high relevance for class separability) in the vicinity of such features. Some of the relevant image features might be as small as tens of micrometres. More generally, any information available prior to the creation of an optimized (second) grid may be used, including but not limited to, NIR-light imaging and already performed dermatofluoroscopic scans on the given sample. Further preferred, the first ML algorithm and the second ML algorithm may be part of a common ML algorithm or may form distinct algorithms.

According to an embodiment combinable with any of the preceding embodiments, a class separability of clusters is defined by: determining image features defined by a brightness value and/or the at least one color value, preferably for grid points of previously scanned grids; pooling the image features to form clusters on basis of a similarity score; analysing, for each of the clusters, the spectra measured in the cluster with the malignancy model so that a class-conditional malignancy score distribution is predicted for each cluster; and determining class separability for each cluster as the overlap of two class-conditional distributions.

Image features (possibly including all other available information) are pooled together to form clusters on the basis of similarity. Clusters are preferably predefined by using large amounts of (historic) image data. Preferably, a state-of-the-art clustering algorithm can be used for this purpose. The spectra measured on a cluster are analysed with a ML model so that class-conditional malignancy score distributions can be predicted for a given image patch. Class separability is defined as the overlap of the two class-conditional distributions. Preferably, spectra are measured on clusters/areas with high predicted class separability. In that regard, a person skilled in the art will recognise that image features may be obtained by using deep learning processing methods such as Super Point, D2-Net and LF-Net or using traditional processing techniques such as Scale Invariant Feature Transform (SIFT), Speed up Robust Feature (SURF) technique, Binary Robust Independent Elementary Features (BRIEF) or Oriented FAST and Rotated BRIEF (ORB).

In an embodiment, the step of determining the second grid comprises (iteratively) adding or removing at least one grid point for individual clusters in order to increase overall class separability of the clusters, i.e., of the clusters of the lesion. This is aimed at minimizing the risk of false negative evaluations and false positive evaluations.

In an embodiment, the final grid points of the second grid, corresponding to each cluster are preferably distributed evenly for the respective clusters. This is aimed at simplifying decision making on where for a given cluster new grid points are to be positioned. For each cluster no location (potential grid point) shall be preferred over others. By creating the second grid, there are less grid points of obtaining TPF spectra in poorly separating areas, which can be understood as a source of noise in data so that reducing the number of the scan points in such areas will improve the Signal-to-Noise (S/N) ratio of the measurement of the lesion of interest.

As an example, a scan density $d_i$ for a particular cluster i may be calculated as the number of scan points to be assigned to the cluster divided by the maximum number of potential scan points available in this cluster (maximum density, $d_i$, of 1 corresponds to scanning all points in the cluster):

$$d_i = optimized_scan_points_in_cluster_i \,/\, max_scan_points_in_cluster_i$$

From this number, the points in the image corresponding to the cluster are preferably filled evenly according to the calculated density, i.e. the density yields a cluster dependent step-size between filling grid points of one cluster:

$$step_i = 1/d_i$$

In an embodiment, the number of grid points of a cluster may be below a predefined maximum threshold and above a predefined minimum threshold number. Such thresholds allow the system not to go outside of limits that aim at avoiding making judgements based on too few scan points, and a maximum threshold, which provides no further reduction of said risk but instead improves usability of the method in constraining the measurement time.

According to an embodiment combinable with any of the preceding embodiments concerning clusters, the overall class-conditional distribution of the lesion is calculated as a weighted average, the weight of each cluster being the proportion of the second grid points in said cluster to the total number of second grid points. This in practice means that changing the number of scan points in a cluster affects the score distributions via the weight (its contribution to the overall predicted score distributions). Some identified clusters correspond to a much higher class separability, so they are predominantly occupied in contrast to other, more noisy clusters.

According to an embodiment combinable with any of the preceding embodiments concerning clusters, the removal or addition of at least one grid point for individual clusters is executed iteratively. However, there are preferably fewer final points in the second grid than in the first grid and a removal of a grid point is not necessarily directly linked to an addition of another point. Preferably, the final aim of this step is to arrive at a very high class separability that minimises any possible false positive or false negative predictions. Thus, a final (second) grid for a scan with high probability of a definitive evaluation is predicted.

A second aspect of the present invention is a method for obtaining a grid model and/or a look up table, LUT, the method comprising: obtaining a polarized dermoscopy, PD, image of a region of interest comprising a skin lesion, wherein each pixel of the PD image comprises a brightness value and/or at least one color value; obtaining, for each of a plurality of grid points of the PD image, a likelihood of detecting a low-noise two photon fluorescence, TPF, spectra; and determining the grid model and/or the LUT, each of both associating image features of defined by a brightness value and/or at least one color value with a likelihood of detecting a low-noise TPF spectra. Herein the PD image refers to historic images. Such images may concern a region of interest comprising a lesion as described according to the first aspect of the present disclosure. The grid points of the PD image may refer herein to the first grid that in preferred embodiments is a regular grid. The likelihood determined here as the grid model and/or the LUT will later be used for inferring the second grid according to the first aspect of the present invention.

Advantageously, after the process according to the present disclosure has been calibrated, i.e., after the grid model and/or the LUT have been determined according to said second aspect of the disclosure, the grid prediction model is enabled to recognize known image features ("clusters"), associate them with a likelihood of detecting low-noise TPF spectra and provide the optimized scan grid for optimal class separability under time constraints according to the first aspect.

According to an embodiment of the second aspect of the disclosure, the method further comprises the steps of: obtaining, for each of the plurality of grid points of the PD image, TPF spectra data; obtaining, for each of the plurality of grid points of the PD image, class separability data; and determining the grid model by associating, for each of the plurality of grid points of the PD image, the PD image data and the class separability data. According to this aspect the calibration step may also use data obtained during the inference of the second grid as well as the final TPF scan data as obtained according to the first aspect of the present disclosure. Preferably, the second grid inference procedure is carried out with an aid of an optimization algorithm (e.g. Gradient Descent), which iteratively removes and/or adds scan/grid points to image areas of known clusters, whereby the expected class separability is changed. An associated loss function (optimization goal) is a function of the overall predicted class separability of the lesion of interest.

According to an embodiment of the second aspect of the disclosure, for each of the plurality of grid points of the PD image, the class separability data is obtained by using a malignancy model that is trained by a (first) machine learning, ML, algorithm and that associates the TPF spectra data, with a class label related to malignancy.

Another aspect of the present invention is a computer program product comprising instructions which, when the program is executed by a computer, cause the computer to carry out the steps of the method of the first aspect of the present invention.

Another aspect of the present invention is a computer program product comprising instructions which, when the program is executed by a computer, cause the computer to carry out the steps of the method of the second aspect of the present invention.

Yet another aspect of the present invention is a computer device comprising a processor configured to carry out the steps of the method of the first aspect of the present invention.

Yet further aspect of the present invention is a computer device comprising a processor configured to carry out the steps of the method of the second aspect of the present invention.

Additionally, an object of the present disclosure is a computer readable medium storing computer-executable instructions performing all the steps of the computer-implemented method according the method of the first aspect of the present invention when executed on a computer.

Additionally, an object of the present disclosure is a computer readable medium storing computer-executable instructions performing all the steps of the computer-implemented method according the method of the second aspect of the present invention when executed on a computer.

A computer-readable (storage) medium, such as referred to herein, typically may be non-transitory and/or comprise a non-transitory device. In this context, a non-transitory storage medium may include a device that may be tangible, meaning that the device has a concrete physical form, although the device may change its physical state. Thus, for example, non-transitory refers to a device remaining tangible despite a change in state.

An example of a non-transitory medium is a non-volatile memory, for example a flash memory while an example of a volatile memory is RAM.

Further aspects of the present invention could be learned from the dependent claims or the following description.

## BRIEF DESCRIPTION OF THE DRAWINGS

These and other objects of the invention presented herein, are accomplished by providing a method for obtaining dermatofluoroscopy data. Further details and features of the present invention, its nature and various advantages will become more apparent from the following detailed description of the preferred embodiments shown in a drawing, in which:

| | |
|---|---|
| Figures 1A to 1C | schematically illustrate an exemplary first and second grids; |
| Figures 2A to 2C | schematically illustrate another exemplary first and second grids; |
| Figures 3A to 3B | schematically illustrate examples of the second grids; |
| Figure 4 | shows the first aspect of the present invention; |
| Figures 5A to 5C | presents a high-level diagram of the processes according to the present invention; |
| Figure 6 | shows an example of original and optimized class separability according to the present disclosure; and |
| Figure 7 | depicts a second aspect of the present invention. |

## DESCRIPTION OF THE DRAWINGS

Reference will now be made in detail to embodiments which are illustrated in the drawings. Effects and features of the exemplary embodiments will be described with reference to the accompanying drawings. Therein, like reference numerals denote like elements, and redundant descriptions are omitted. The present invention, however, may be embodied in various different forms, and should not be construed as being limited to only the illustrated embodiments herein. Rather, these embodiments are provided solely as examples for fully conveying the aspects and features of the present invention to those skilled in the art.

Accordingly, processes, elements, and techniques that are not considered necessary to those having ordinary skill in the art for a complete understanding of the aspects and features of the present invention may not be described. At the same time, within the drawings, the relative sizes of elements, layers, and regions may be exaggerated for clarity.

As used herein, the term "and/or" includes any and all combinations of one or more of the associated listed items. Further, the use of "may" when describing embodiments of the present invention refers to "one or more embodiments of the present invention." Further, in the following description of embodiments of the present invention, the terms of a singular form may include plural forms unless the context clearly indicates otherwise.

It will be understood that although the terms "first" and "second" are used to describe various elements, these elements should not be limited by these terms. These terms are only used to distinguish one element from another element. For example, a first element may be named a second element and, similarly, a second element may be named a first element, without departing from the scope of the present invention. As used herein, the term "and/or" includes any and all combinations of one or more of the associated listed items and expressions such as "at least one of" when preceding a list of elements, modify the entire list of elements.

As used herein, terms such as "substantially" and "about" are used as terms of approximation and not as terms of degree, and are intended to account for the inherent deviations in measured or calculated values that would be recognized by those of ordinary skill in the art. However, if the term "substantially" is used in combination with a feature expressed using a numeric value, the term "substantially" denotes a range of +/- 5% of the value centered on the value.

Figures 1A to 1C schematically illustrate exemplary first and second grids.

According to Figure 1A a skin lesion 103 is located in a region of interest 101 where the method according to the present disclosure is to be applied. Figure 1B shows that the first grid 102 is applied to the region of interest 101. The first grid 102 is in this example uniformly spaced and may be considered an initial or a coarse grid set for the purpose of mapping to a PD image. Figure 1C presents the second grid 102B where the first grid points 102 are optimized wherein said optimization preferably results in fewer second grid points (less dense arrangement) in one or more areas 104A identified with lower class-separability or higher noise (usually areas of lower interest) and in more dense arrangement grid points in one or more areas 104B identified as malignant with high risk and/or as having higher class-separability or less noise (areas of high interest).

Figures 2A to 2C schematically illustrate another exemplary first and second grids.

In this example it is depicted that an area 105 surrounded by skin lesion 103, i.e., an "inner part" of lesion 103, may also be an area of interest with additional grid points. In this embodiment, area 105 and lesion 103 have a different appearance. This does not necessarily imply that area 105 is less likely to be malignant than lesion 103. However, area 105 has another class separability than skin lesion 103, particularly a lower class separability than skin lesion 103. Nevertheless by increasing a number of obtained TPF spectra in a region of lesion 103 as well as in area 105, potentially improves class separability overall, i.e., for the region of interest as a whole.

Figures 3A to 3B schematically illustrate examples of the second grids 102B wherein it is shown that the area 104B having an increased number of second grid points 102B may have the points distributed evenly as shown in Figure 3A or differently than evenly as shown in Figure 3B.

According to the examples of the optimized scan grid for dermatofluoroscopy depicted in Figures 1A to 3B, a pigmented lesion of interest is suspected of melanoma and should be measured with dermatofluoroscopy. With the standard grid (Figure 1B), the spectroscopic measurement of the entire lesion would take about 30 minutes. An improved grid (Figure 1C) not only enables a faster scan but also potentially improves the diagnostic accuracy of the method by focusing on the pixel clusters (pooled according to the image features) associated with a high signal-to-noise ratio defined by class separability (higher scanning raster resolution in relevant areas, lower resolution in insignificant areas). The procedure of scanning with optimized densities requires high accuracy in the positioning of the optics that deliver laser light (fiber) to the relevant image clusters due to potentially high density of scan points required. This is preferably achieved with a linear delta robot. The linear delta robot operation converts the movements of three parallel linear actuators into three purely translational movements (x, y, z) of its end effector. This is preferably achieved through the use of parallelogram kinematics.

Figure 4 shows the first aspect of the present invention as a process diagram.

Accordingly, the method for obtaining dermatofluoroscopy data, comprises the steps of: illuminating S401 a skin lesion using a predetermined light spectrum (the skin lesion (103) may be present in an illuminated region of interest (101)); obtaining S402 a polarized dermoscopy, PD, image using a camera, wherein each pixel of the PD image comprises a brightness value and/or at least one color value; defining S403 a first grid and mapping the first grid to the PD image; determining S404, for each grid point of the first grid, based on the brightness value and/or the at least one color value, a first likelihood of detecting low-noise two photon fluorescence, TPF, spectra in the vicinity of the grid point; determining S405 a second grid based on the determined first likelihoods for the first grid points; performing S406 a dermatofluoroscopic scan, including the detection of TPF spectra, on the grid points of the second grid; and determining S407 a second likelihood for each grid point of the second grid based on the dermatofluoroscopic scan, the second likelihood being associated with malignancy.

Figure 5 presents a high-level diagram of the processes according to the present invention.

The diagrams shown in Figures 5A to 5C are high-level block diagrams referring to the first and second aspect of the present disclosure. The first aspect of the present disclosure relates to Grid Inference 505 and Improved Classification after a physical scan is performed 509. These aspects are shown in Figures 5B and 5C, where in Figure 5B an image (the PD image) 506 is input to a grid decision module 507, which based on the aforementioned process identifies the second grid 102B and outputs the second grid 102B once it has been finally determined 508. At this stage the grid decision module 507 refers to the second grid 102B and identifying the second likelihoods.

Correspondingly, Figure 5C refers to the first aspect of the present disclosure in that spectra values from the second grid as obtained 510 with the TPF scan are submitted to a classification model 511 in order to determine sample classes at given points and pixel features such as color, brightness or image features.

The second aspect of the present invention is shown in Figure 5A where a Calibration / Training 501 phase is schematically depicted. Image of a PD 502 is input together with 503 spectra values, exemplarily measured at a first grid 102 for training a grid decision module 504 that correlates the likelihood of a low-noise spectrum 503 with certain image features of the PD 502.

Figure 6 show an example of original and optimized class separability according to the present disclosure.

Two graphs 601A and 601B shown in Figure 6 depict an example of the presently disclosed optimization procedure on a melanoma sample, originally identified as false negative. The class separability is optimized by allocating or removing scan points resulting in altered predicted class-conditional distributions. The measured score 604 and decision boundary/cut-off 605 are visualized both for the original, first grid 601A and for the optimized second grid 601B. Since the data was collected on a regular first grid, the measured score line 604 in the optimized graph 601B is a prospective projection (extrapolation). Note that the lesion was classified correctly only with an optimized grid 601B. The cut-off 605 boundary is preferably adjusted to generate a high sensitivity classification. The cut-off may be preferably placed such as to generate a high sensitivity classification to avoid false negative results (in the illustrated case at the far left end of the melanoma-conditioned distribution 601B and 605). The threshold separates the melanoma-conditioned distribution into predicted true positive and false negative ranges. The area under false negative range of the distribution is to be minimized. The MM abbreviation in Figure 6 stands for a melanoma while the corresponding nMM stands for non-melanoma.

Figure 7 depicts the second aspect of the present invention.

The second aspect of the present invention is a method for obtaining a grid model and/or a look up table, LUT, the method comprising: obtaining a polarized dermoscopy, PD, image of a skin lesion S701 (or a PD image of a region of interest (101) comprising a skin lesion (103)), wherein each pixel of the PD image comprises a brightness value and/or at least one color value; obtaining S702, for each of a plurality of grid points of the PD image, a likelihood of detecting a low-noise two photon fluorescence, TPF, spectra; and determining S703 the grid model and/or the LUT, each of both associating image features of defined by a brightness value and/or at least one color value with a likelihood of detecting a low-noise TPF spectra.

According to an optional embodiment of the second aspect of the disclosure, the method further comprises the steps of: obtaining S704, for each of the plurality of grid points of the PD image, TPF spectra data; obtaining S705, for each of the plurality of grid points of the PD image, class separability data; and determining S706 the grid model by associating, for each of the plurality of grid points of the PD image, the PD image data and the class separability data.

The electronic or electric devices and/or any other relevant devices or components according to embodiments of the present invention described herein, except those described explicitly as hardware, may be implemented utilizing any suitable hardware, firmware (e.g. an application-specific integrated circuit), software, or a combination of software, firmware, and hardware. For example, the various components of these devices may be formed on one integrated circuit (IC) chip or on separate IC chips.

Further, the various components of these devices may be implemented on a flexible printed circuit film, a tape carrier package (TCP), a printed circuit board (PCB), or formed on one substrate. The electrical connections or interconnections described herein may be realized by wires or conducting elements, e.g. on a PCB or another kind of circuit carrier. The conducting elements may comprise metallization, e.g. surface metallization and/or pins, and/or may comprise conductive polymers or ceramics. Further electrical energy might be transmitted via wireless connections, e.g. using electromagnetic radiation and/or light.

Further, the various components of these devices may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like.

A person of skill in the art should recognize that the functionality of various computing devices may be combined or integrated into a single computing device, or the functionality of a particular computing device may be distributed across one or more other computing devices without departing from the scope of the exemplary embodiments of the present invention. Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly defined so.

## REFERENCE SIGNS

101 region of interest
102 first grid
102B second grid
103 skin lesion
104A area identified as non-malignant with a relatively high probability
104B area identified as malignant with a relatively high probability
105 area identified as non-malignant with a relatively high probability, having a denser grid
S401 to S407 Method steps
501 calibration / training process
502 image data
503 spectra values in the first grid
504 grid decision module
505 grid Inference process
506 an image

507 grid decision module
508 optimal, second grid for spectra measurement
509 improved classification process
510 spectra values from the optimum, second grid
511 classification model
512 identified sample class
601A example graph of poor class separability
601B example graph of good class separability
602 predicted score distribution for melanoma measurements
603 predicted score distribution for non-melanoma measurements
604 measured score value
605 cut off boundary
S701 to S706 Method steps

**Claims**

1. A method for obtaining dermatofluoroscopy data, the method comprising:

illuminating (S401) a region of interest (101) comprising a skin lesion (103) using a predetermined light spectrum;
obtaining (S402) a polarized dermoscopy, PD, image using a camera, wherein each pixel of the PD image comprises a brightness value and/or at least one color value;
defining (S403) a first grid (102) and mapping the first grid (102) onto the PD image;
determining (S404), for each grid point of the first grid, based on the brightness value and/or the at least one color value, a first likelihood of detecting low-noise two photon fluorescence, TPF, spectra in the vicinity of the grid point;
determining (S405) a second grid (102B) based on the determined first likelihoods for the first grid (102) points;
performing (S406) a dermatofluoroscopic scan, including the detection of TPF spectra, on the grid points of the second grid (102B); and
determining (S407) a second likelihood for each grid point of the second grid (102B) based on the dermatofluoroscopic scan, the second likelihood being associated with malignancy.

2. The method of claim 1, wherein the second likelihood is determined using a malignancy model trained by a machine learning, ML, algorithm and associating dermatofluoroscopic scan data, including at least TPF spectra data, with a class label related to malignancy.

3. The method of claim 2, wherein a low-noise TPF spectrum provides a class separability above a predefined threshold when input in said malignancy model, said class separability being related to at least two classes of mutually exclusive class labels.

4. The method of any one of the above claims, wherein the first likelihood is determined by a grid model and/or a look up table, LUT, each of both associating image features defined by a brightness value and/or the at least one color value with a likelihood of detecting a low-noise TPF spectra.

5. The method of claim 4, wherein the grid model is determined in a calibration phase based on PD image data, TPF spectra data and class separability data.

6. The method of claim 5, wherein the grid model is trained in the calibration phase by a machine learning, ML, algorithm associating, for each grid point of a plurality of grids on which dermatofluoroscopic scans were previously performed, PD image data, TPF spectra data and class separability data.

7. The method of any one the preceding claims, wherein a class separability of clusters is defined by:

determining image features defined by a brightness value and/or the at least one color value;
pooling the image features to form clusters on basis of a similarity score;
analysing, for each of the clusters, the spectra measured in the cluster with the malignancy model so that a class-conditional score distribution is predicted for each cluster; and
determining class separability for each cluster as the overlap area of class-conditional distributions between classes.

**8.** The method of claim 7, wherein the step of determining the second grid (102B) comprises iteratively adding or removing at least one grid point for individual clusters in order to increase overall class separability of the clusters .

**9.** The method of claim 8, wherein the final grid points of the second grid, corresponding to each cluster, are distributed evenly for the respective clusters.

**10.** The method of claims 8 or 9, wherein the number of grid points of a cluster is below a predefined maximum threshold and above a predefined minimum threshold number.

**11.** The method of any one of claims 7 to 10, wherein the lesion is assigned its own overall class-conditional distribution calculated as a weighted average, the weight of each cluster being the proportion of the second grid points for said cluster to the total number of all second grid points for the lesion.

**12.** A method for obtaining a grid model and/or a look up table, LUT, the method comprising:

obtaining (S701) a polarized dermoscopy, PD, image of a region of interest (101) comprising a skin lesion (103), wherein each pixel of the PD image comprises a brightness value and/or at least one color value;
obtaining (S702), for each of a plurality of grid points of the PD image, a likelihood of detecting a low-noise two photon fluorescence, TPF, spectra; and
determining (S703) the grid model and/or the LUT, each of both associating image features of defined by a brightness value and/or at least one color value with a likelihood of detecting a low-noise TPF spectra.

**13.** The method of claim 12, further comprising the steps of:

obtaining (S704), for each of the plurality of grid points of the PD image, TPF spectra data;
obtaining (S705), for each of the plurality of grid points of the PD image, class separability data; and
determining (S706) the grid model by associating, for each of the plurality of grid points of the PD image, the PD image data and the class separability data.

**14.** The method of claim 13, wherein, for each of the plurality of grid points of the PD image, the class separability data is obtained by using a malignancy model that is trained by a machine learning, ML, algorithm and that associates the TPF spectra data, with a class label related to malignancy.

**15.** A computer-readable medium comprising instructions which, when executed by a computer, cause the computer to carry out the method of any of claims 1 to 14.

**Amended claims in accordance with Rule 137(2) EPC.**

**1.** A method for obtaining dermatofluoroscopy data, the method comprising:

illuminating (S401) a region of interest (101) comprising a skin lesion (103) using a predetermined light spectrum;
obtaining (S402) a polarized dermoscopy, PD, image using a camera, wherein each pixel of the PD image comprises a brightness value and/or at least one color value;
defining (S403) a first grid (102) and mapping the first grid (102) onto the PD image;
determining (S404), for each grid point of the first grid, based on the brightness value and/or the at least one color value, a first likelihood of detecting low-noise two photon fluorescence, TPF, spectra in the vicinity of the grid point;
determining (S405) a second grid (102B) based on the determined first likelihoods for the first grid (102) points, wherein the second grid (102B) comprises less grid points than the first grid (102A) in one or more areas (104A) identified with lower class-separability or higher noise;
performing (S406) a dermatofluoroscopic scan, including the detection of TPF spectra, on the grid points of the second grid (102B).

**2.** The method of claim 1, wherein the method further comprises a step of determining (S407) a second likelihood for each grid point of the second grid (102B) based on the dermatofluoroscopic scan, the second likelihood being associated with malignancy, wherein the second likelihood is determined using a malignancy model trained by a machine learning, ML, algorithm and associating dermatofluoroscopic scan data, including at least TPF spectra data, with a class label related to malignancy.

**3.** The method of claim 2, wherein a low-noise TPF spectrum provides a class separability above a predefined threshold when input in said malignancy model, said class separability being related to at least two classes of mutually exclusive class labels.

**4.** The method of any one of the above claims, wherein the first likelihood is determined by a grid model and/or a look up table, LUT, each of both associating image features defined by a brightness value and/or the at least one color value with a likelihood of detecting a low-noise TPF spectra.

**5.** The method of claim 4, wherein the grid model is determined in a calibration phase based on PD image data, TPF spectra data and class separability data.

**6.** The method of claim 5, wherein the grid model is trained in the calibration phase by a machine learning, ML, algorithm associating, for each grid point of a plurality of grids on which dermatofluoroscopic scans were previously performed, PD image data, TPF spectra data and class separability data.

**7.** The method of any one the preceding claims, wherein a class separability of clusters is defined by:

determining image features defined by a brightness value and/or the at least one color value;
pooling the image features to form clusters on basis of a similarity score;
analysing, for each of the clusters, the spectra measured in the cluster with the malignancy model so that a class-conditional score distribution is predicted for each cluster; and
determining class separability for each cluster as the overlap area of class-conditional distributions between classes.

**8.** The method of claim 7, wherein the step of determining the second grid (102B) comprises iteratively adding or removing at least one grid point for individual clusters in order to increase overall class separability of the clusters.

**9.** The method of claim 8, wherein the final grid points of the second grid, corresponding to each cluster, are distributed evenly for the respective clusters.

**10.** The method of claims 8 or 9, wherein the number of grid points of a cluster is below a predefined maximum threshold and above a predefined minimum threshold number.

**11.** The method of any one of claims 7 to 10, wherein the lesion is assigned its own overall class-conditional distribution calculated as a weighted average, the weight of each cluster being the proportion of the second grid points for said cluster to the total number of all second grid points for the lesion.

**12.** The method according to claim 4 wherein the grid model and/or a look up table, LUT, are obtained by:

obtaining (S701) a polarized dermoscopy, PD, image of a region of interest (101) comprising a skin lesion (103), wherein each pixel of the PD image comprises a brightness value and/or at least one color value;
obtaining (S702), for each of a plurality of grid points of the PD image, a likelihood of detecting a low-noise two photon fluorescence, TPF, spectra; and
determining (S703) the grid model and/or the LUT, each of both associating image features of defined by a brightness value and/or at least one color value with a likelihood of detecting a low-noise TPF spectra.

**13.** The method of claim 12, further comprising the steps of:

obtaining (S704), for each of the plurality of grid points of the PD image, TPF spectra data;
obtaining (S705), for each of the plurality of grid points of the PD image, class separability data; and
determining (S706) the grid model by associating, for each of the plurality of grid points of the PD image, the PD image data and the class separability data.

**14.** The method of claim 13, wherein, for each of the plurality of grid points of the PD image, the class separability data is obtained by using a malignancy model that is trained by a machine learning, ML, algorithm and that associates the TPF spectra data, with a class label related to malignancy.

**15.** The method of claim 1 wherein the second grid (102B) comprises a lower number of scan points than the first grid (102A).

**16.** A computer-readable medium comprising instructions which, when executed by a computer, cause the computer to carry out the method of any of claims 1 to 15.

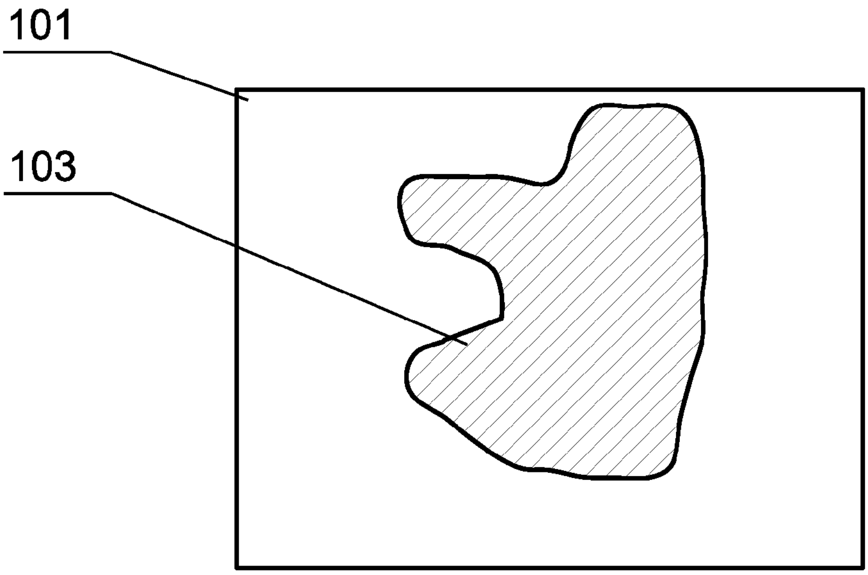


Fig. 1A

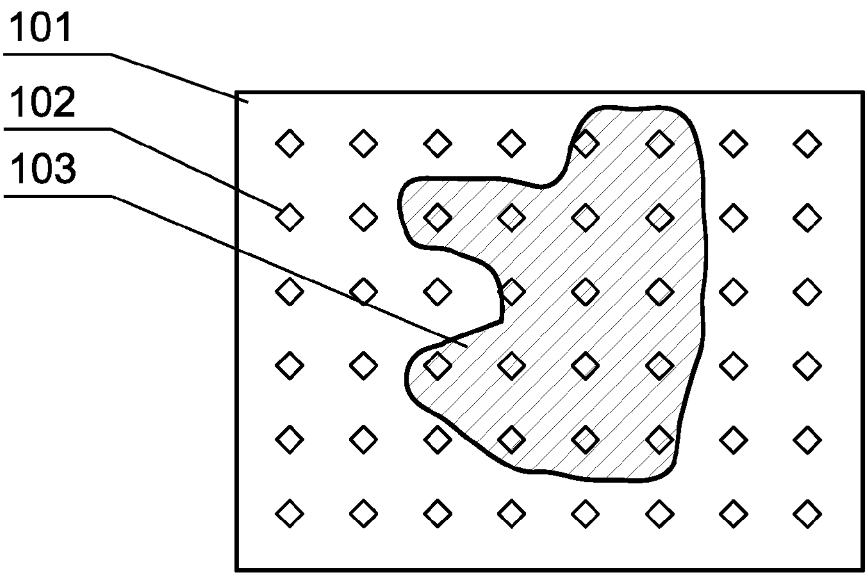


Fig. 1B

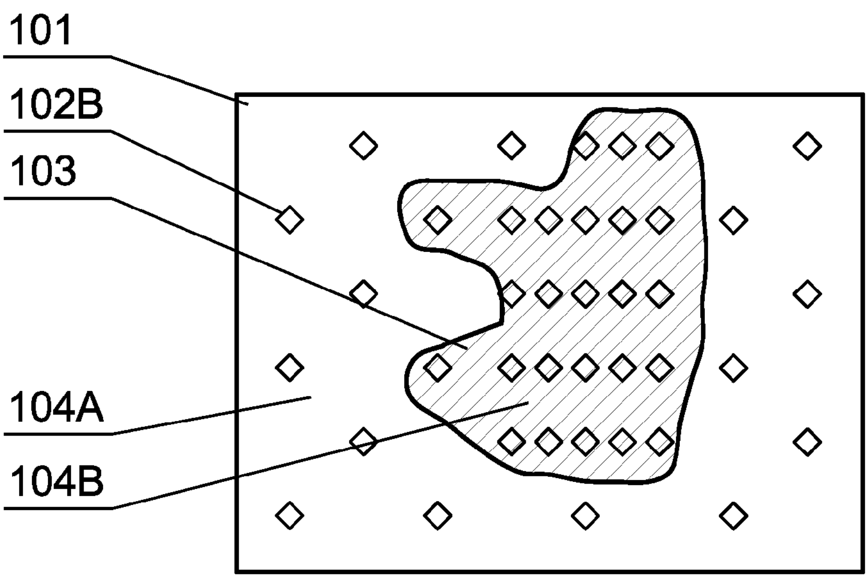


Fig. 1C

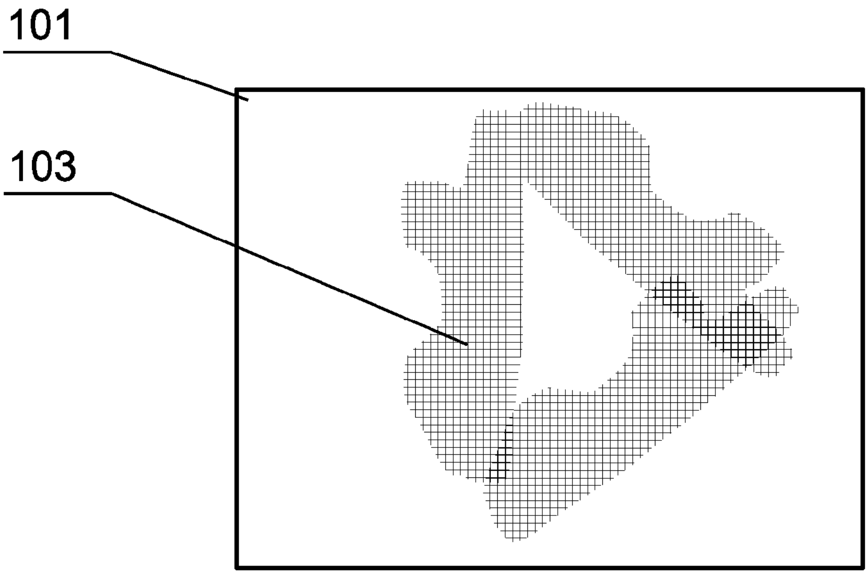


Fig. 2A

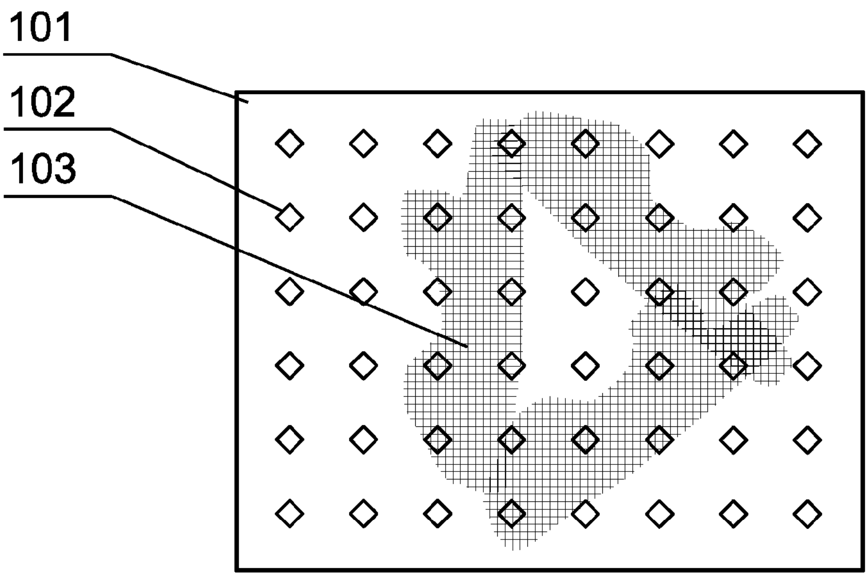


Fig. 2B

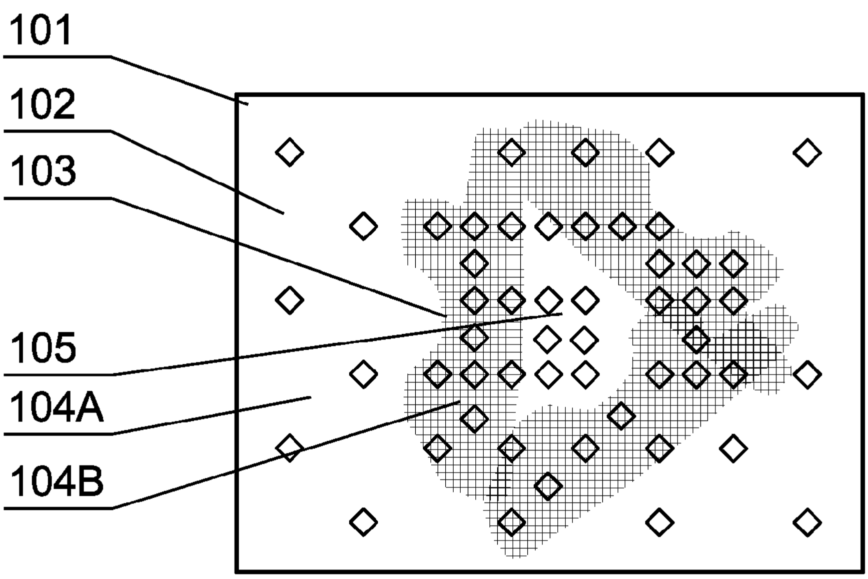


Fig. 2C

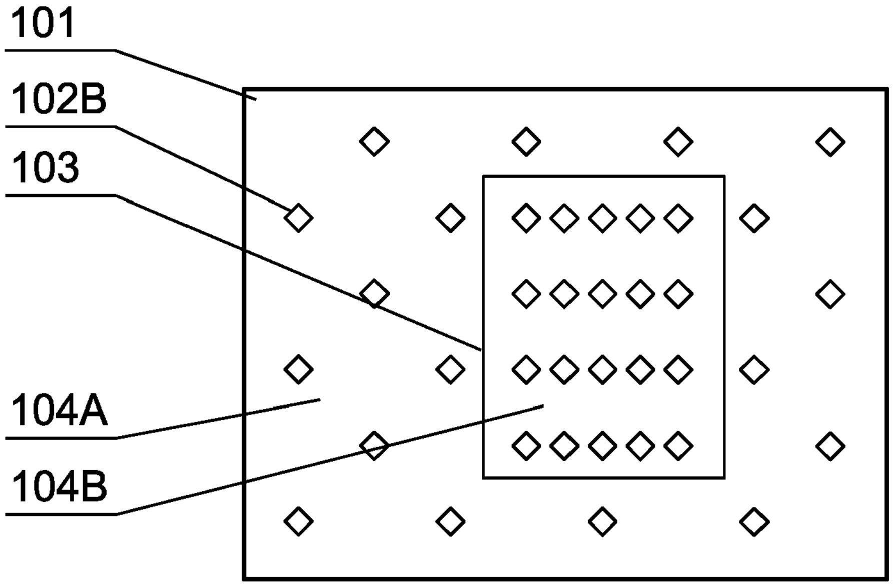


Fig. 3A

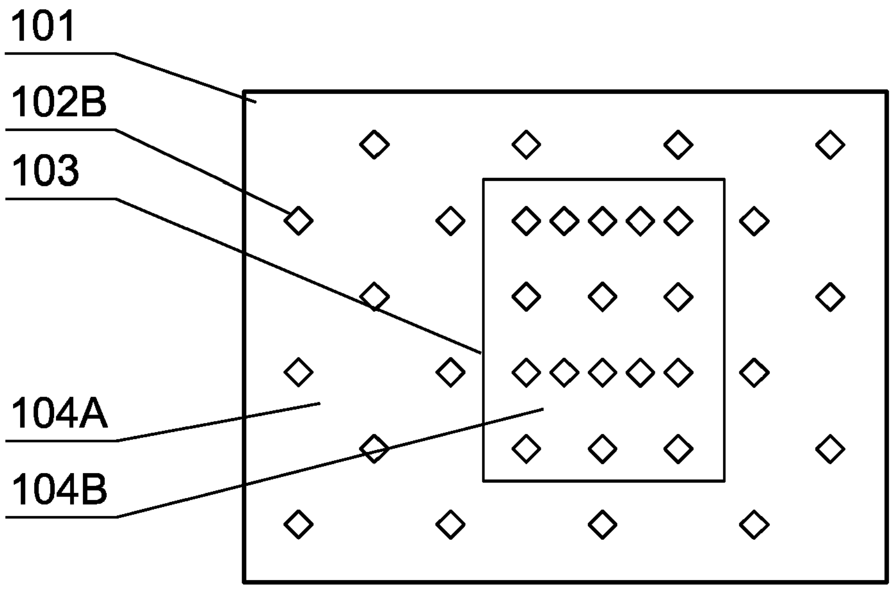


Fig. 3B

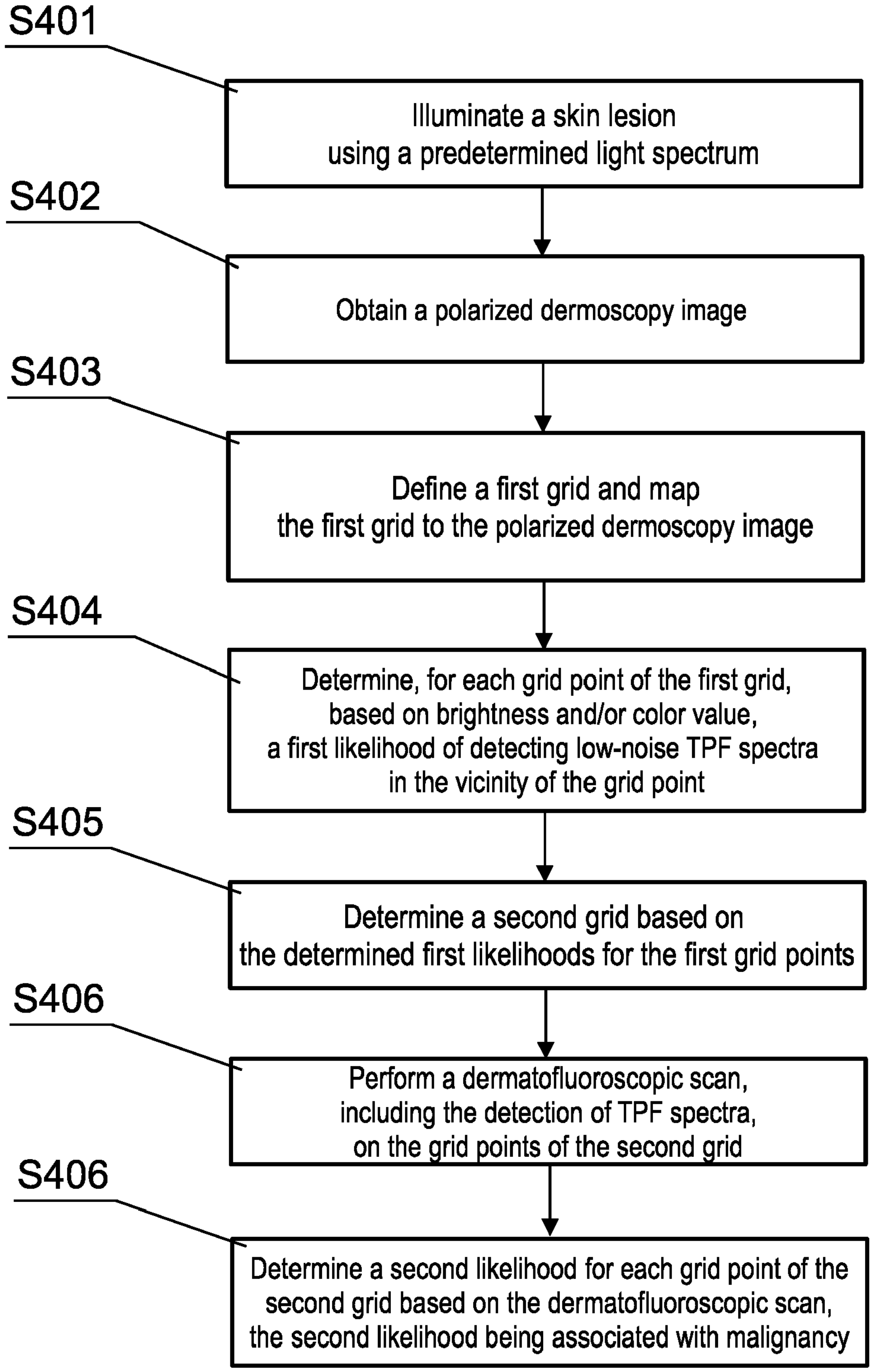
S401
Illuminate a skin lesion using a predetermined light spectrum
S402
Obtain a polarized dermoscopy image
S403
Define a first grid and map the first grid to the polarized dermoscopy image
S404
Determine, for each grid point of the first grid, based on brightness and/or color value, a first likelihood of detecting low-noise TPF spectra in the vicinity of the grid point
S405
Determine a second grid based on the determined first likelihoods for the first grid points
S406
Perform a dermatofluoroscopic scan, including the detection of TPF spectra, on the grid points of the second grid
S406
Determine a second likelihood for each grid point of the second grid based on the dermatofluoroscopic scan, the second likelihood being associated with malignancy

Fig. 4

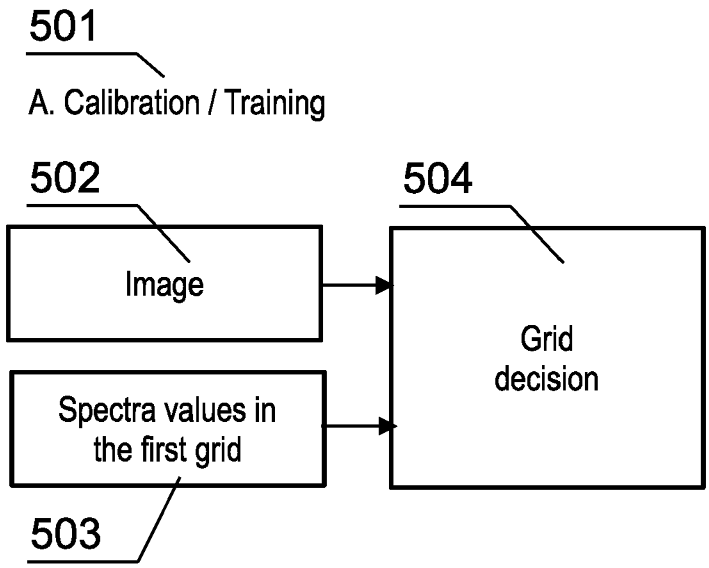


Fig. 5A

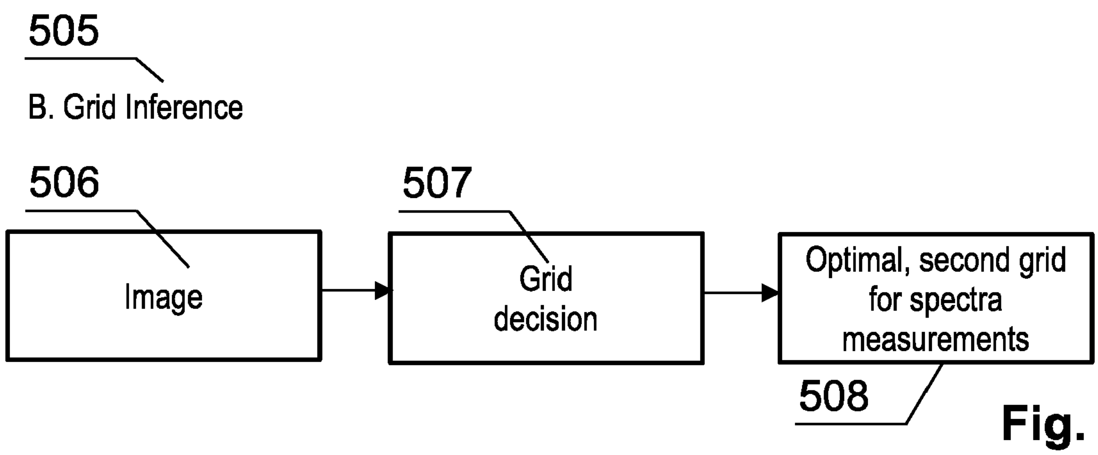


Fig. 5B

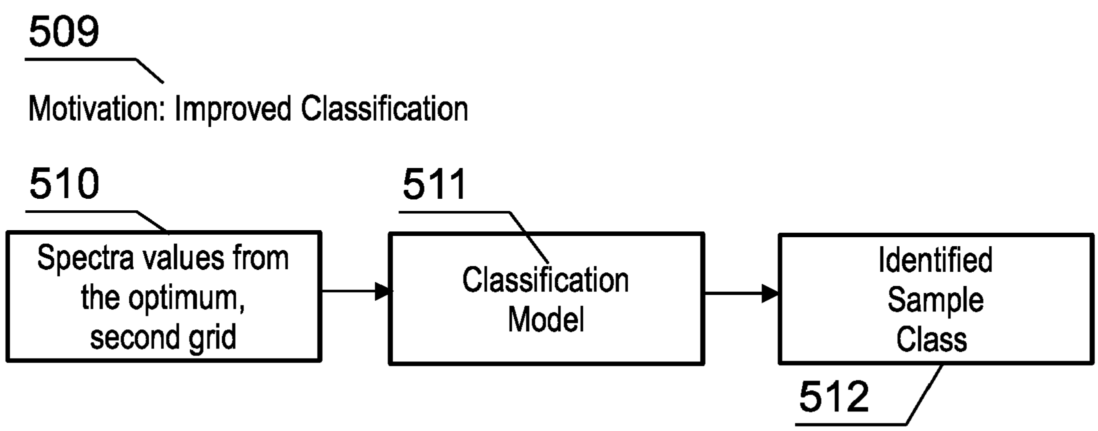


Fig. 5C

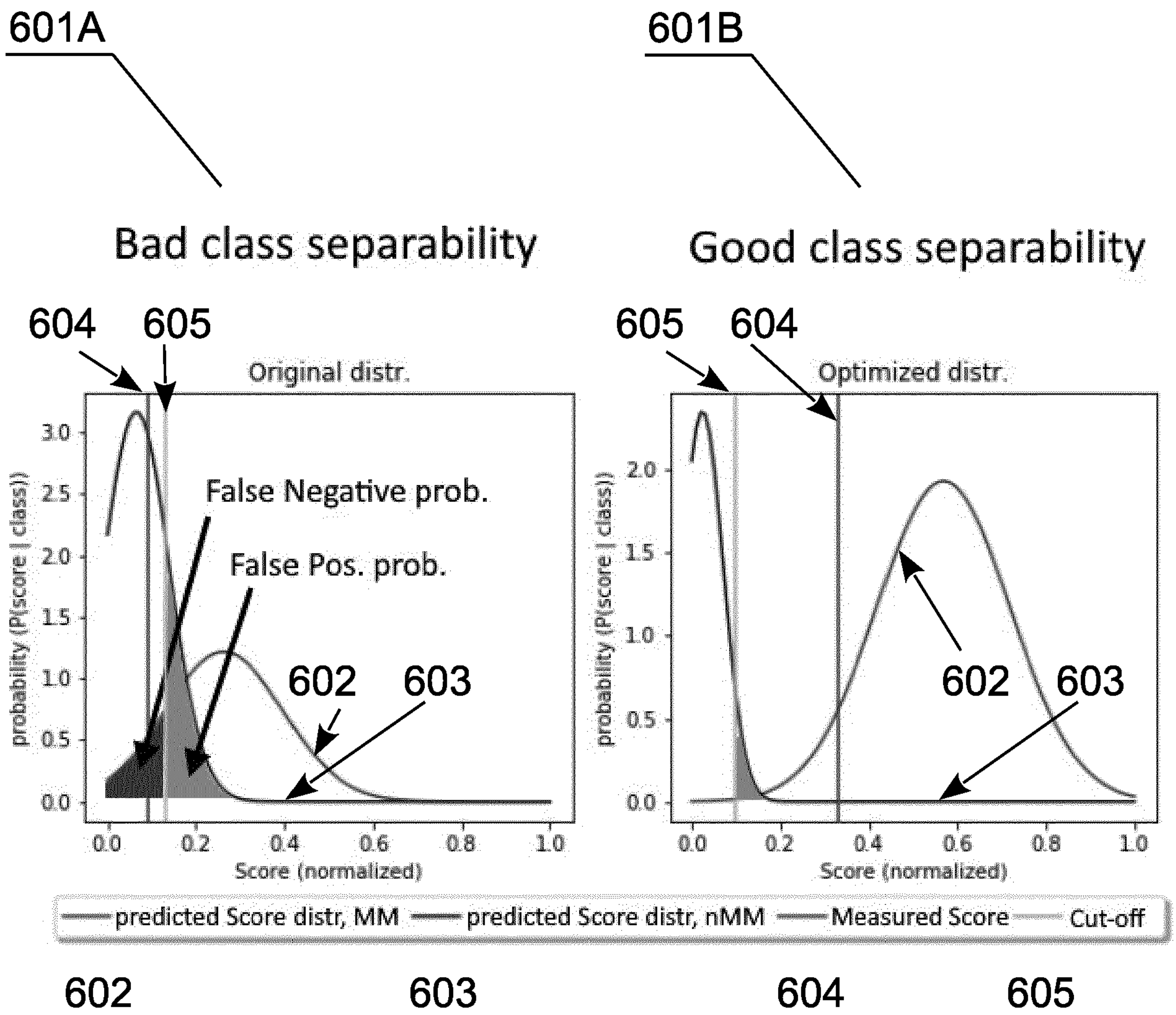
601A
601B
Bad class separability
Good class separability
604
605
605
604
Original distr.
Optimized distr.
False Negative prob.
False Pos. prob.
602
603
602
603
probability (P(score | class))
Score (normalized)
predicted Score distr, MM
predicted Score distr, nMM
Measured Score
Cut-off
602
603
604
605

Fig. 6

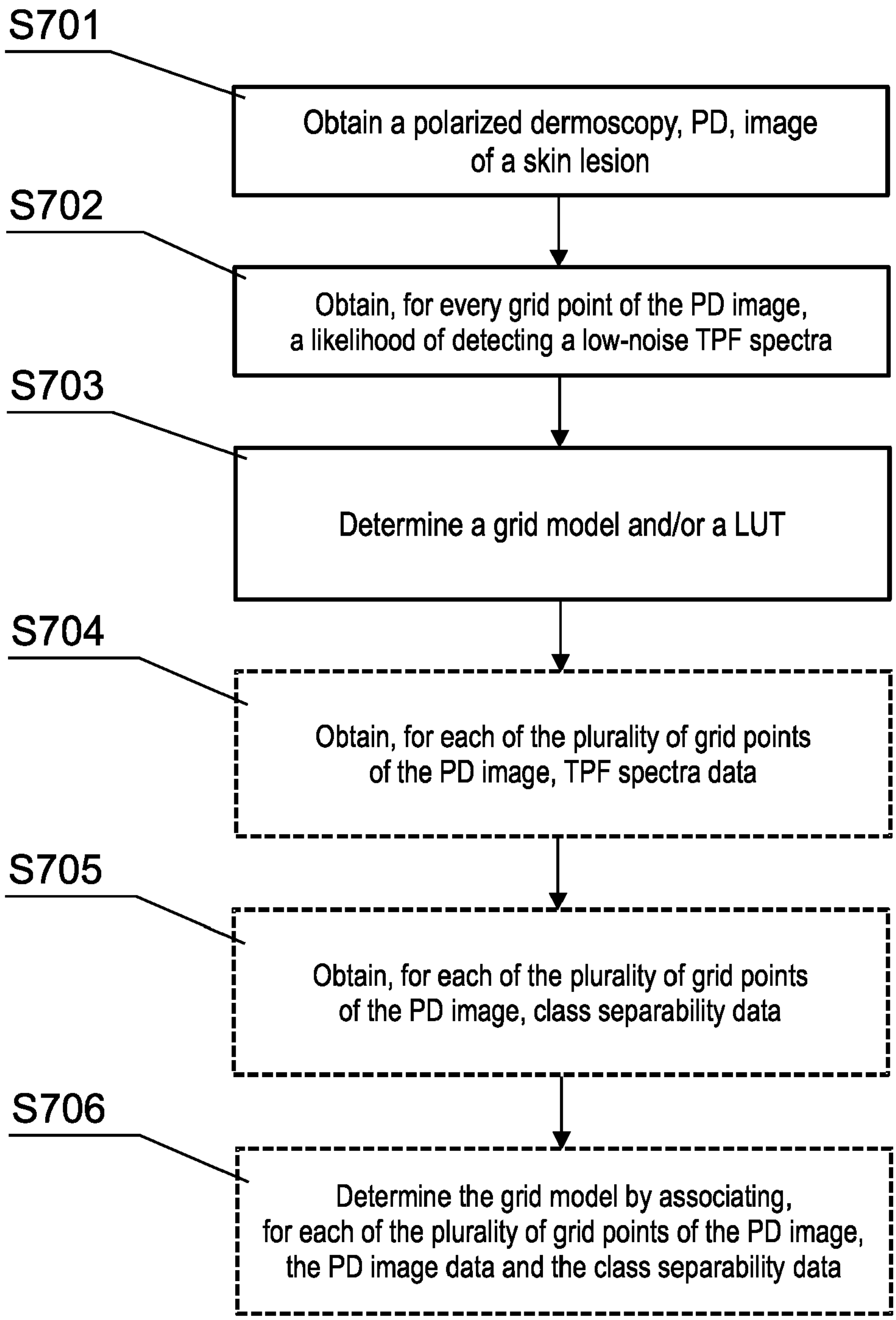
S701
Obtain a polarized dermoscopy, PD, image of a skin lesion
S702
Obtain, for every grid point of the PD image, a likelihood of detecting a low-noise TPF spectra
S703
Determine a grid model and/or a LUT
S704
Obtain, for each of the plurality of grid points of the PD image, TPF spectra data
S705
Obtain, for each of the plurality of grid points of the PD image, class separability data
S706
Determine the grid model by associating, for each of the plurality of grid points of the PD image, the PD image data and the class separability data

Fig. 7

Europäisches Patentamt
European Patent Office
Office européen des brevets

# EUROPEAN SEARCH REPORT

Application Number
EP 22 18 7471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEUPOLD DIETER ET AL: "The stepwise two-photon excited melanin fluorescence is a unique diagnostic tool for the detection of malignant transformation in melanocytes : Melanin fluorescence as diagnostic tool",<br>PIGMENT CELL & MELANOMA RESEARCH, [Online] vol. 24, no. 3, 2 May 2011 (2011-05-02), pages 438-445, XP093009585,<br>United States, Denmark<br>ISSN: 1755-1471, DOI: 10.1111/j.1755-148X.2011.00853.x<br>Retrieved from the Internet:<br>URL:https://onlinelibrary.wiley.com/doi/epdf/10.1111/j.1755-148X.2011.00853.x><br>[retrieved on 2022-12-19]<br>* abstract *<br>* sect. "Significance";<br>page 438 *<br>* page 441, right-hand column, paragraph 3 *<br>* figures 4A-B, 5, 6 * | 1-15 | INV.<br>A61B5/00<br>A61B5/103<br>G06T7/90<br>G01N21/00 |
| A | & EICHHORN REINHOLD ET AL: "Early diagnosis of melanotic melanoma based on laser-induced melanin fluorescence",<br>JOURNAL OF BIOMEDICAL OPTICS,<br>vol. 14, no. 3,<br>1 January 2009 (2009-01-01), page 034033, XP093009589,<br>1000 20th St. Bellingham WA 98225-6705 USA<br>ISSN: 1083-3668, DOI: 10.1117/1.3155511<br>Retrieved from the Internet:<br>URL:https://www.spiedigitallibrary.org/journalArticle/Download?urlId=10.1117%2F1.3155511><br>[retrieved on 2022-12-19]<br>* the whole document *<br>-----<br>-/-- | 1-15 | TECHNICAL FIELDS SEARCHED (IPC)<br>A61B<br>G01N<br>G06T |

The present search report has been drawn up for all claims

1

| Place of search | Date of completion of the search | Examiner |
|---|---|---|
| The Hague | 11 January 2023 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 6

Europäisches Patentamt
European Patent Office
Office européen des brevets

# EUROPEAN SEARCH REPORT

Application Number
EP 22 18 7471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MACKINNON NICHOLAS ET AL: "Toward diagnosis of skin cancer using multimode imaging dermoscopy: (I) clinical system development and validation", PROGRESS IN BIOMEDICAL OPTICS AND IMAGING, SPIE - INTERNATIONAL SOCIETY FOR OPTICAL ENGINEERING, BELLINGHAM, WA, US, [Online] vol. 8947, 4 March 2014 (2014-03-04), pages 89470I-89470I, XP060033841, ISSN: 1605-7422, DOI: 10.1117/12.2041818 ISBN: 978-1-5106-0027-0 Retrieved from the Internet: URL:https://www.spiedigitallibrary.org/proceedings/Download?urlId=10.1117%2F12.2041818> [retrieved on 2022-12-19] * abstract * * sect.4 "Conclusion" * * figures 1,2 * ----- -/-- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 11 January 2023 | Delval, Christophe |

1

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

page 2 of 6

Europäisches Patentamt
European Patent Office
Office européen des brevets

# EUROPEAN SEARCH REPORT

Application Number
EP 22 18 7471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FORSCHNER A ET AL: "Diagnostic accuracy of dermatofluoroscopy in cutaneous melanoma detection: results of a prospective multicentre clinical study in 476 pigmented lesions", BRITISH JOURNAL OF DERMATOLOGY, JOHN WILEY, HOBOKEN, USA, [Online] vol. 179, no. 2, 7 June 2018 (2018-06-07), pages 478-485, XP071167181, ISSN: 0007-0963, DOI: 10.1111/BJD.16565 Retrieved from the Internet: URL:https://academic.oup.com/bjd/article-pdf/179/2/478/47218347/bjd0478.pdf> [retrieved on 2022-12-19] * abstract * * sect. "Investigational device and diagnostic algorithm" * * figures 1,2 * * tables 1,2 * ----- | 1-15 | |
| X | SZYC LUKASZ ET AL: "Supervised learning with low-quality ground truth in early diagnostics of malignant melanoma", SPIE PROCEEDINGS; [PROCEEDINGS OF SPIE ISSN 0277-786X], SPIE, US, [Online] vol. 11469, 20 August 2020 (2020-08-20), pages 114690A-114690A, XP060133116, DOI: 10.1117/12.2571475 ISBN: 978-1-5106-3673-6 Retrieved from the Internet: URL:https://www.spiedigitallibrary.org/proceedings/Download?urlId=10.1117%2F12.2571475> [retrieved on 2022-12-19] * abstract * * sect.2-4 * * sect.4.2 * * figures 2,3 * ----- | 1-15 | TECHNICAL FIELDS SEARCHED (IPC) |

-/--

The present search report has been drawn up for all claims

1

| Place of search | Date of completion of the search | Examiner |
|---|---|---|
| The Hague | 11 January 2023 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 18 7471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ASHTAMI K M ET AL: "Classification of Melanoma on Dermoscopy Images using SVM Classifier", INTERNATIONAL JOURNAL FOR RESEARCH IN APPLIED SCIENCE AND ENGINEERING TECHNOLOGY, [Online] vol. 6, no. 4, 1 April 2018 (2018-04-01), pages 2631-2638, XP093009495, DOI: 10.22214/ijraset.2018.4440 Retrieved from the Internet: URL:https://www.ijraset.com/fileserve.php?FID=16198> [retrieved on 2022-12-19] * abstract * * sect.V; figures 1-3 * ----- | 1-15 | |
| A | KEERTHIKA T ET AL: "A Color Based Approach to Detect Melanoma Using SVM Classifier", 2022 INTERNATIONAL CONFERENCE ON ADVANCED COMPUTING TECHNOLOGIES AND APPLICATIONS (ICACTA), IEEE, [Online] 4 March 2022 (2022-03-04), pages 1-5, XP034113119, DOI: 10.1109/ICACTA54488.2022.9752921 Retrieved from the Internet: URL:https://ieeexplore.ieee.org/stamp/stamp.jsp?tp=&arnumber=9752921> [retrieved on 2022-12-19] * abstract * * figures 1-4 * * the whole document * ----- | 1-15 | TECHNICAL FIELDS SEARCHED (IPC) |

-/--

The present search report has been drawn up for all claims

1

| Place of search | Date of completion of the search | Examiner |
|---|---|---|
| The Hague | 11 January 2023 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 18 7471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SZYC LUKASZ ET AL: "Diagnostic Performance of a Support Vector Machine for Dermatofluoroscopic Melanoma Recognition: The Results of the Retrospective Clinical Study on 214 Pigmented Skin Lesions", DIAGNOSTICS, [Online] vol. 9, no. 3, 25 August 2019 (2019-08-25), page 103, XP093009508, DOI: 10.3390/diagnostics9030103 Retrieved from the Internet: URL:https://www.mdpi.com/2075-4418/9/3/103/pdf?version=1566728105> [retrieved on 2022-12-19] * abstract * * figures 2-5 * * the whole document * ----- | 1-15 | |
| A | US 2009/224172 A1 (SCHOLZ MATTHIAS [DE] ET AL) 10 September 2009 (2009-09-10) * abstract * * claims 14-22 * * figures 1A-B, 2, 3B, 4A-B * ----- | 1-15 | TECHNICAL FIELDS SEARCHED (IPC) |

-/--

The present search report has been drawn up for all claims

| Place of search | Date of completion of the search | Examiner |
|---|---|---|
| The Hague | 11 January 2023 | Delval, Christophe |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 5 of 6

European Patent Office

# EUROPEAN SEARCH REPORT

Application Number
EP 22 18 7471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WINKLER JULIA K ET AL: "Association between different scale bars in dermoscopic images and diagnostic performance of a market-approved deep learning convolutional neural network for melanoma recognition", EUROPEAN JOURNAL OF CANCER, ELSEVIER, AMSTERDAM NL, [Online] vol. 145, 16 January 2021 (2021-01-16), pages 146-154, XP086505979, ISSN: 0959-8049, DOI: 10.1016/J.EJCA.2020.12.010 Retrieved from the Internet: URL:https://www.sciencedirect.com/science/article/pii/S0959804920314209/pdfft?md5=e9bcd52ccd8556158a2b5bf88a2e39ed&pid=1-s2.0-S0959804920314209-main.pdf> [retrieved on 2022-12-19] * abstract * * the whole document * * sect.4 * * figures 1,3,4 * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 11 January 2023 | Delval, Christophe |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 6 of 6

# ANNEX TO THE EUROPEAN SEARCH REPORT ON EUROPEAN PATENT APPLICATION NO.

EP 22 18 7471

This annex lists the patent family members relating to the patent documents cited in the above-mentioned European search report. The members are as contained in the European Patent Office EDP file on
The European Patent Office is in no way liable for these particulars which are merely given for the purpose of information.

11-01-2023

| Patent document cited in search report | Publication date | Patent family member(s) | Publication date |
|---|---|---|---|
| US 2009224172 A1 | 10-09-2009 | AU 2007264252 A1 | 03-01-2008 |
| | | CA 2654417 A1 | 03-01-2008 |
| | | DE 102006029809 B3 | 08-11-2007 |
| | | EP 2032970 A1 | 11-03-2009 |
| | | US 2009224172 A1 | 10-09-2009 |
| | | WO 2008000223 A1 | 03-01-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7888659 B2